# EUROPEAN PATENT SPECIFICATION

(11) **EP 2 647 724 B1**
(45) Date of publication and mention of the grant of the patent: **08.03.2017**
(21) Application number: 11844878.6
(22) Date of filing: 25.11.2011
(51) Int. Cl.: C12Q 1/68, C12N 15/12

(54) **METHOD FOR SCREENING A SUBSTANCE FOR PREVENTING SKIN AGING**
VERFAHREN ZUM SCREENING VON SUBSTANZEN ZUR VERHINDERUNG VON HAUTALTERUNG
PROCÉDÉ DE CRIBLAGE DES AGENTS POUR LA PRÉVENTION DU VIEILLISSEMENT DE LA PEAU

(30) Priority: 30.11.2010 KR 20100120571
(43) Date of publication of application: 09.10.2013
(73) Proprietor: Amorepacific Corporation, Seoul 140-777 (KR); SNU R & DB Foundation, Seoul 151-818 (KR)
(72) Inventor: SON, Eui Dong, Yongin-si Gyeonggi-do 446-580 (KR); LEE, Jin Young, Yongin-si Gyeonggi-do 446-783 (KR); NA, Yong Ju, Yongin-si Gyeonggi-do 446-902 (KR); KIM, Hyae Kyoung, Seongnam-si Gyeonggi-do 463-870 (KR); KIM, Ji Hyun, Yongin-si Gyeonggi-do 446-905 (KR); AHN, Soo Mi, Suwon-si Gyeonggi-do 443-757 (KR); KIM, Han Kon, Suwon-si Gyeonggi-do 443-793 (KR); CHUNG, Jin Ho, Seoul 137-060 (KR); PARK, Chi Hyun, Ansan-si Gyeonggi-do 425-906 (KR); SEO, Eun Young, Seoul 137-060 (KR); LEE, Min Jung, Seoul 136-753 (KR); OH, Inn Gyung, Seoul 150-102 (KR); YOON, Hyun Sun, Seoul 140-713 (KR)
(74) Representative: Ilgart, Jean-Christophe
(86) International application number: PCT/KR2011/009057
(87) International publication number: WO 2012/074246

(56) References cited:
- WO-A2-02/090934
- WO-A2-2009/048282
- JP-A- 2005 520 483
- JP-A- 2007 259 851
- KR-A- 20080 057 233
- KR-A- 20090 021 786
- KR-A- 20090 036 299
- KR-A- 20090 051 573
- KR-A- 20100 051 286
- LENER T ET AL: "Expression profiling of aging in the human skin", EXPERIMENTAL GERONTOLOGY, ELSEVIER SCIENCE, OXFORD, GB, vol. 41, no. 4, 1 April 2006 (2006-04-01), pages 387-397, XP027937029, ISSN: 0531-5565 [retrieved on 2006-04-01]
- AOKI H ET AL: "Gene expression profiling analysis of solar lentigo in relation to immunohistochemical characteristics", BRITISH JOURNAL OF DERMATOLOGY, OXFORD : WILEY-BLACKWELL, UK , vol. 156, no. 6 1 June 2007 (2007-06-01), pages 1214-1223, XP009119932, ISSN: 0007-0963, DOI: 10.1111/J.1365-2133.2007.07830.X Retrieved from the Internet: URL:http://www3.interscience.wiley.com/cgi -bin/issn?DESCRIPTOR=PRINTISSN&VALUE=0007- 0963
- ROBINSON MK ET AL: "Genomic-driven insights into changes in aging skin", JOURNAL OF DRUGS IN DERMATOLOGY, STRATEGIC COMMUNICATION IN DERMATOLOGY, NEW YORK, NY, US, vol. 8, no. 7, suppl, 1 July 2009 (2009-07-01), pages s8-s11, XP009175371, ISSN: 1545-9616
- DAVID B ALLISON ET AL: "Microarray data analysis: from disarray to consolidation and consensus", NATURE REVIEWS: GENETICS, NATURE PUBLISHING GROUP, UNITED KINGDOM, vol. 7, no. 1, 1 January 2006 (2006-01-01) , pages 55-65, XP009100551, ISSN: 1471-0064, DOI: 10.1038/NRG1749

## Description

### Technical Field

The present invention relates to a method for screening substances which prevent skin aging by controlling the expression of genes showing characteristic changes associated with skin aging.

### Background Art

Until now, studies on skin aging have been conducted mainly on changes in the dermal layer of the skin and have been focused on the regulation of biosynthesis of collagen and the collagenase MMP (matrix metalloproteinase). Also, the only active ingredient in anti-aging cosmetic products, which is known to be effective in increasing collagen biosynthesis, is retinol.

However, the prevention of skin aging cannot be sufficiently achieved by a simple increase in collagen synthesis, and skin aging symptoms and related factors differ according to various causes of aging. Thus, a new approach which comprehensively considers various factors is required for the development of next-generation functional cosmetic products. In addition, it is required to ascertain the causes of skin aging by comprehensively analyzing characteristic genetic changes associated with skin aging and understand the mechanisms of skin aging, thereby developing technologies for preventing and treating skin aging, which control the expression of target genes associated with skin aging.

The international application WO02/090934proposes a marker gene set comprising genes the ression of which is altered following exposure to UV radiation. The identification of these genes performed on human skin samples and in particular on human keratomes has been carried out usine total RNA and a microarray and by analyzine the results with ImageauaNT™ software. The International application WO02/090934 also proposes to implement this set of genes to evaluate the UV radiation projective, reparative and/or therapeutic effects of a compound or material.

### Disclosure

### Technical Problem

Accordingly, the present inventors have made extensive efforts to find novel target genes related to aging prevention by comprehensively screening genes, which show characteristic changes associated with skin aging, using gene chips.

Therefore, herein disclosed are new target genes related to aging prevention.

The object of the present invention is to provide a method for screening novel substances, which show the effects of inhibiting skin aging and treating skin diseases by controlling the expression of the above genes.

### Technical Solution

In order to accomplish the above objects, the present disclosure provides a method for screening a gene related to skin aging, the method comprising the steps of: 1) extracting total RNA from skin tissue and subjecting the extracted RNA to microarray analysis; 2) analyzing genes, which change with aging, using a bioinformatics technique; 3) selecting a candidate gene related to skin aging; and 4) confirming the candidate gene by reverse transcription-polymerase chain reaction (RT-PCR).

The present invention concerns a method for screening a substance for preventing skin aging, the method comprising the steps of: 1) treating sample skin cells with a candidate substance for controlling the expression of a gene in the cells; 2) exposing the sample skin cells to an environment having a skin aging factor to induce the expression of the gene in the cells; and 3) measuring the skin aging factor-induced expression of the gene in the cells of step 2), as defined in claim 1.

### Advantageous Effects

Genes related to skin aging can be screened according to the various causes of skin aging. Further, not only genes related to photoaging caused by UV radiation, but also genes causing intrinsic aging associated with menopause, can be investigated, and differentiated products can be developed according to target genes. Thus, skin aging can be fundamentally prevented.

### Description of Drawings

FIG. 1 shows the results of microarray analysis and quantitative RT-PCR performed on galanin prepropeptide in the skins of young people and elderly people.
FIGS. 2 and 3 show the results obtained by inhibiting the expression galanin prepropeptide by the siRNA method and then measuring the UV-induced change in the expression of anti-aging markers (MMP) and inflammatory cytokines in order to analyze the function of galanin prepropeptide.
FIG. 4 shows the relationship between the expressions of galanin prepropeptide and collagen, determined by inhibiting the expression of galanin prepropeptide using a method similar to the method described for FIGS. 2 and 3.
FIG. 5 shows the results of microarray analysis and quantitative RT-PCR performed on osteoglycin in the skins of young people and elderly people.
FIG. 6 shows the relationship between the expressions of galanin prepropeptide and collagen, determined by inhibiting the expression of osteoglycin.
FIG. 7 shows 16 genes whose expression increased in seborrheic keratosis.

### Best Mode

Although skin aging is caused by various factors, the present invention will be described with a focus on the following aspects.
1. Intrinsic aging caused by the passage of time
2. Menopause aging caused by hormones
3. Seborrheic keratosis

The present disclosure provides a method of screening genes related to aging, caused by various factors, by performing microarray analysis.

The present disclosure provides a method for screening a gene related to skin aging, the method comprising the steps of: 1) extracting total RNA from skin tissue and subjecting the extracted RNA to microarray analysis; 2) analyzing genes, which change with aging, using a bioinformatics technique; 3) selecting a candidate gene related to skin aging; and 4) confirming the candidate gene by reverse transcription-polymerase chain reaction (RT-PCR).

Genes which are involved or not involved in skin aging, or genes whose expression increases or decreases in men and women, differ depending on the causes of skin aging, and the genes whose expression changes can be determined as genes related to skin aging. Thus, such genes can be selected as candidate genes related to skin aging.

The present inventors have found by the screening method that galanin prepropeptide, Wnt3, TNS4, osteoglycin and the like can be novel target gene related to skin aging.

The present disclosure provides a method for screening substances capable of preventing skin aging by controlling the expression of the above target genes.

Candidate substances effective in preventing skin aging can be screened by searching using a cell search system and then evaluating the effects on the human body using the skin of volunteers.

The present invention also provides a method for screening a substance for preventing skin aging, the method comprising the steps of: 1) treating sample skin cells with a candidate substance for controlling the expression of a gene in the cells; 2) exposing the sample skin cells to an environment having a skin aging factor to induce the expression of the gene in the cells; and 3) measuring the skin aging factor-induced expression of the gene in the cells of step 2), as defined in claim 1.

As the sample cells in step 1) of the method of the present invention, any cells may be used, as long as they can induce the expression of skin aging-related genes therein when being subjected to an environment which causes skin aging. Examples of the environment having the skin aging factor include, but are not limited to, various factors causing skin aging, including UV rays, heat, and hormones.

In step 3) of the method of the present invention, the skin aging factor-induced expression of the gene in the cells can be measured by reverse transcription-polymerase chain reaction (RT-PCR).

According to the above-described method, it is possible to find novel target genes related to skin aging and develop new and differentiated products that act on skin aging-related genes according to the various causes of skin aging-related genes.

### Mode for Invention

Hereinafter, the present invention will be described in further detail with reference to examples. It is to be understood, however, that these examples are illustrative purposes only

### Test Example 1: Microarray analysis for intrinsic aging model (not within the scope of the present invention)

In order to screen genes related to intrinsic skin aging caused by the passage of time, a test was carried out on human skin.

18-25 years old young people (4 men and 4 women) and 78-82 years old elderly people (4 men and 4 women) were selected as subjects, and 4 to 6 buttock skin samples were collected from each of the subjects by a 4-8 mm punch biopsy.

Total RNA was extracted from the collected skin samples and subjected to microarray analysis (Affymetrix GeneChip Human HG-U133 Plus2.0), and genes which changed with aging were analyzed using a bioinformatics technique (DAVID Bioinformatics Resources/Gene spring).

Candidate genes related to skin aging were selected based on the results of the analysis and confirmed by quantitative RT-PCR.

Table 1 below shows the number of genes whose expression increased or decreased in men or women.

**Table 1**

| | Present only in men | Common | | Present only in women |
|---|---|---|---|---|
| | | Men | Women | |
| Total | 497 | 200 | 200 | 123 |
| Increased | 405 | 38 | 38 | 56 |
| Decreased | 92 | 162 | 162 | 67 |

| | | | | |
|---|---|---|---|---|
| (2-fold changed U133-2 probes with p<=0.05, No FDR) | | | | |

Because there were no probes that increased in men but decreased in women, there was no probe having a difference between men and women in common genes.

Table 2 below shows genes whose expression increased in the skins of both elderly men and elderly women.

**Table 2**

| Gene name | Gene symbol |
|---|---|
| Lipoprotein lipase | LPL |
| Adiponectin Adiponectin, C1Q and collagen domain containing | ADIPOQ |
| Phospholamban | PLN |

Table 3 below shows genes whose expression decreased in the skins of both elderly men and elderly women.

**Table 3**

| Gene name | Gene symbol |
|---|---|
| Arachidonate 15-lipoxygenase, type B | ALOX15B |
| Small proline-rich protein 1A | SPRR1A |
| ATPase, H+/K+ transporting, nongastric, alpha polypeptide | STP12A |
| Insulin-induced gene 1 | INSIG1 |
| Galanin prepropeptide | GAL |

Table 4 below shows genes whose expression increased in the skin of elderly men.

**Table 4**

| Gene name | Gene symbol |
|---|---|
| Cyclin-dependent kinase 6 | CDK6 |
| Cyclin D1 | CCND1 |
| Janus kinase 2 (a protein tyrosine kinase) | JAK2 |
| Catenin (cadherin-associated protein), alpha 1, 102 kDa | CONNAZ1 |
| cAMP responsive element binding protein 1 | CREB1 |
| Slit homolog 2 (Drosophila) | SLIT2 |

Table 5 below shows genes whose expression decreased in the skin of elderly men.

**Table 5**

| Gene name | Gene symbol |
|---|---|
| WNT inhibitory factor 1 | WIF1 |
| Defensin, beta 4 | DEFB4 |
| Retinol dehydrogenase 11 (all-trans/9-cis/11-cis) | RDH11 |
| Farnesyl diphosphate synthase (farnesyl pyrophosphate synthetase, dimethylallytranstransferase, geranyltranstransferase) | FDPS |

Table 6 below shows genes whose expression increased in the skin of elderly women.

**Table 6**

| Gene name | Gene symbol |
|---|---|
| Matrix metallopeptidase 3 (stromelysin 1, progelatinase) | MMP3 |
| c-fos induced growth factor (vascular endothelial growth factor D) | FIGF |
| chymase 1, mast cell | CMA1 |
| R-spondin 3 homolog (Xenopus laevis) | RSPO3 |
| Dipeptidyl-peptidase 4 | DPP4 |

Table 7 below shows genes whose decreased in the skin of elderly women.

**Table 7**

| Gene name | Gene symbol |
|---|---|
| Collagen, type I, alpha 1 | COL1A1 |
| Latrophilin 3 | LPHN3 |
| GREB1 protein | GREB1 |
| Osteoglycin | OGN |

In the present invention, galanin prepropeptide (GAL) whose expression decreased in the skins of both elderly men and elderly women was selected as a candidate gene related to skin aging. The galanin prepropeptide gene is produced in the neural cells and non-neural cells during development of the nervous system, and the GAL receptors (GALRI, GALR2 and GALR3) are essential factors in biological functions, including immunity, cell proliferation, inflammatory reactions, responses to drugs and stress, insulin secretion, growth hormone secretion, and the like.

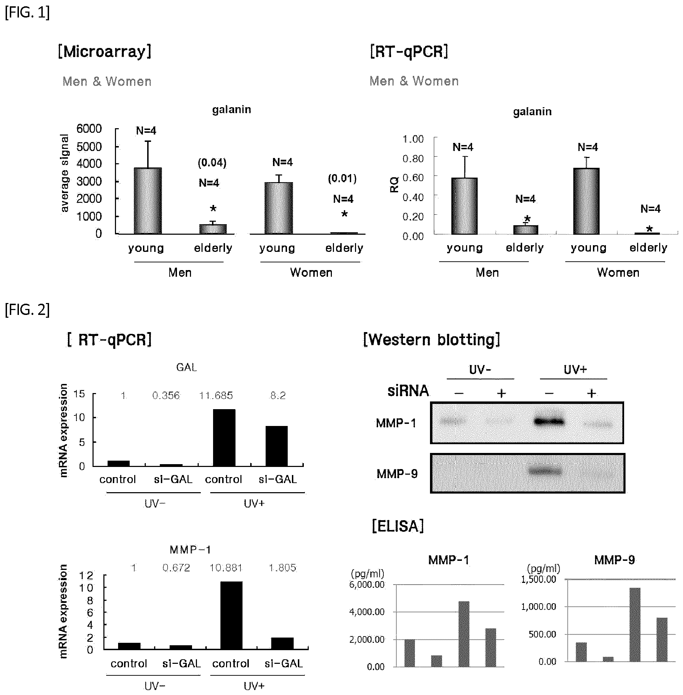

In the present invention, microarray analysis and quantitative RT-PCR were performed for the galanin prepropeptide collected from the skins of young people and elderly people, and the results of the analysis are shown in FIG. 1.

As can be seen in FIG. 1, the results of microarray analysis were consistent with the results of quantitative RT-PCR. Thus, galanin prepropeptide was confirmed as a candidate gene.

The function of galanin prepropeptide was analyzed by the siRNA method.

Specifically, HaCaT cells were dispensed in 60 mm cell culture dishes with 10% serum-containing DMEM medium at a density of 1.25 x 10⁶cells/dish, and then cultured to a confluency of about 80% in a 5% CO₂ incubator at 37°C. The cells were treated with siRNA and incubated, and then the medium was removed. Then, 1 ml of trizol (Invitrogen) was added to the cells, and RNA was isolated from the cells according to the RNA isolation method (Invitrogen). The RNA was quantified using a UV detector (HEWLETT PACKARD) at 260 nm and subjected to RT-PCR (reverse transcription-polymerase chain reaction). For gene analysis for each sample, normalization was performed with respect to the complementary gene 36B4. Also, the ELISA analysis of MMP-1 and MMP-9 was performed according to the method of the manufacture (Amersharm).

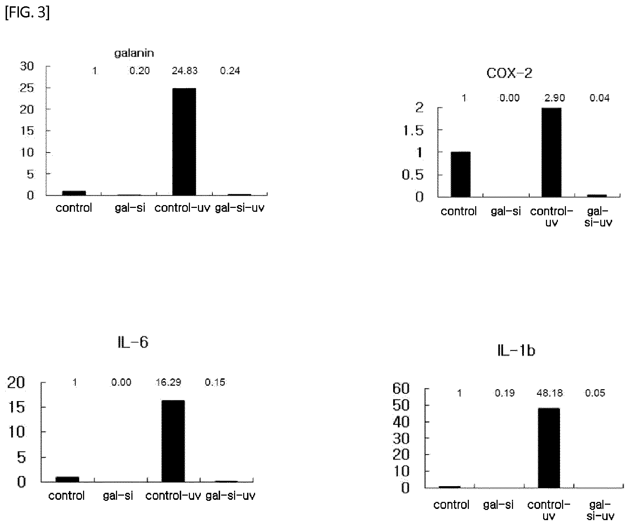

The results of the measurement are shown in FIGS. 2 and 3.

As can be seen in FIG. 2, when skin cells were damaged by UV radiation, the expression of galanin prepropeptide was increased and the expressions of MMP-1 and MMP-9 were also increased.

As can be seen in FIG. 3, UV radiation increased the expression of galanin prepropeptide and also increased the expressions of COX-1, IL-6 and IL-1b.

In addition, the relationship between the expression of galanin prepropeptide and the expression of collagen was examined using a method similar to the above-described method.

Specifically, HaCaT cells were dispensed in 60 mm cell culture dishes with 10% serum-containing DMEM medium at a density of 1.25 x 10⁶cells/dish, and then cultured to a confluency of about 80% in a 5% CO₂ incubator at 37°C. The cells were treated with siRNA and incubated, and then the medium was removed. Then, 1 ml of trizol (Invitrogen) was added to the cells, and RNA was isolated from the cells according to the RNA isolation method (Invitrogen). The RNA was quantified using a UV detector (HEWLETT PACKARD) at 260 nm and subjected to RT-PCR (reverse transcription-polymerase chain reaction). For gene analysis for each sample, normalization was performed with respect to the complementary gene 36B4. Also, the ELISA analysis of procollagen was performed according to the method of the manufacture (Takara).

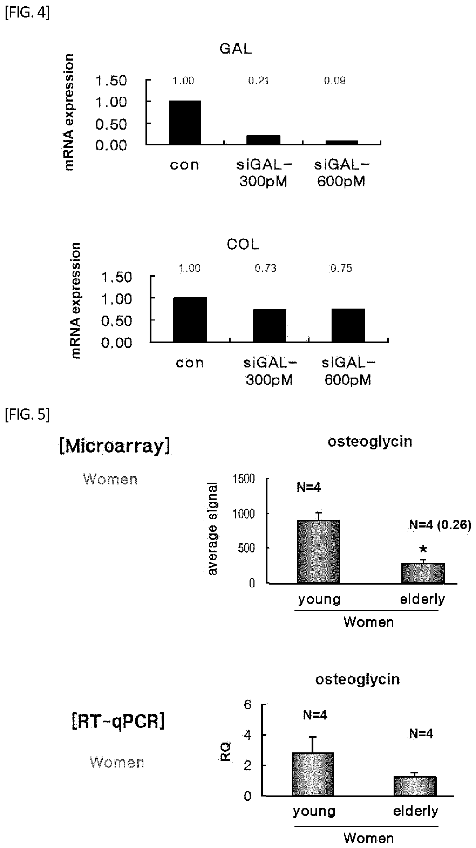

The results of the measurement are shown in FIG. 4.

As can be seen in FIG. 4, when the expression of galanin prepropeptide was insufficient, the expression of collagen also decreased.

### Test Example 2: Microarray analysis for menopause aging model

In this Test Example, the results of Test Example 1 were used. In the present invention, the osteoglycin gene whose expression decreased only in the skin cells of elderly women was selected as a candidate gene. The osteoglycin gene is involved in collagen synthesis, and the expression of osteoglycin decreases, the synthesis of collagen also decreases. Also, osteoglycin has been studied mainly on corneal regeneration and degenerative arthritis and is involved in normal collagen fibrillogenesis, cell growth, cell differentiation, and the recovery of injured tissue.

In the present invention, microarray analysis and quantitative RT-PCR were performed on the osteoglycin collected from young women and elderly women, and the results of the analysis are shown in FIG. 5.

As can be seen in FIG. 5, the results of microarray analysis were consistent with the results of quantitative RT-PCR. Thus, osteoglycin was confirmed as a candidate gene.

The function of osteoglycin was analyzed by the siRNA method.

Specifically, HaCaT cells were dispensed in 60 mm cell culture dishes with 10% serum-containing DMEM medium at a density of 1.25 x 10⁶cells/dish, and then cultured to a confluency of about 80% in a 5% CO₂ incubator at 37°C. The cells were treated with siRNA and incubated, and then the medium was removed. Then, 1 ml of trizol (Invitrogen) was added to the cells, and RNA was isolated from the cells according to the RNA isolation method (Invitrogen). The RNA was quantified using a UV detector (HEWLETT PACKARD) at 260 nm and subjected to RT-PCR (reverse transcription-polymerase chain reaction). For gene analysis for each sample, normalization was performed with respect to the complementary gene 36B4. Also, the ELISA analysis of osteoglycin was performed according to the method of the manufacture (Amersharm).

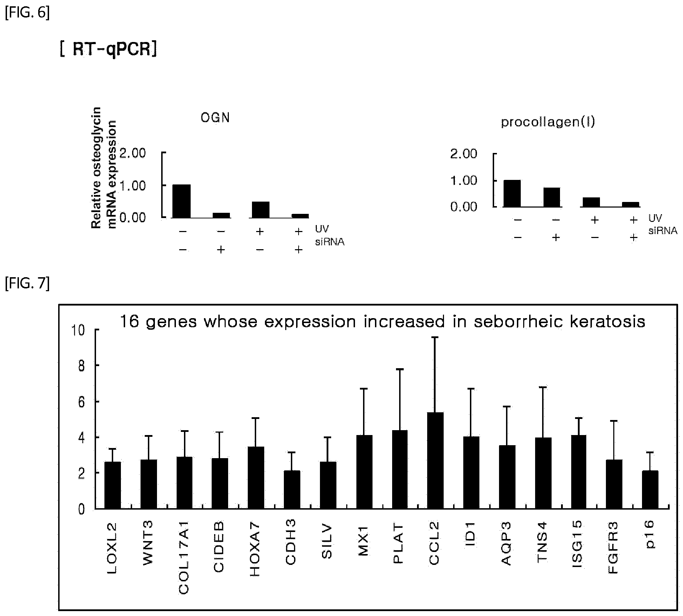

The results of the measurement are shown in FIG. 6.

As can be seen in FIG. 6, the formation of collagen was inhibited in the absence of osteoglycin.

### Test Example 3: Microarray analysis for seborrheic keratosis model (not within the scope of the present invention).

Microarray analysis was performed on seborrheic keratosis and normal skin to compare the expression of genes therebetween.

78-82 years old elderly people (4 men and 4 women) with seborrheic keratosis were selected as subjects, and 4-6 skin samples having seborrheic keratosis and 4-6 skin samples having no seborrheic keratosis were collected from each subject by a 4-8 mm punch biopsy.

Total RNA was extracted from the collected skin samples and subjected to microarray analysis (Affymetrix GeneChip Human HG-U133 Plus2.0), and genes which changed with aging were analyzed using a bioinformatics technique (DAVID Bioinformatics Resources/Gene spring).

16 genes whose expression increased in seborrheic keratosis were determined by the gene analysis and are shown in FIG. 7.

As described above, genes related to skin aging differ depending on the various causes of skin aging. Thus, differentiated products effective in preventing skin aging can be developed by screening substance capable of controlling the expression of these genes.

## Claims

1. An *in vitro* method for screening a substance for preventing skin aging associated with menopause aging, the method comprising the steps of:
1) treating sample skin cells with a candidate substance for controlling the expression of a gene in the cells;
2) exposing the sample skin cells to an environment having a skin aging factor to induce the expression of the gene in the cells;
3) measuring the skin aging factor-induced expression of the gene in the cells of step 2); and
4) identifying the candidate substance as substance for reventin skin ageing if it controls the expression of a gene in the cells,
wherein said gene is OGN,
wherein the expression of OGN is decreased in the skin of elderly women when compared to young women.

## Patentansprüche

1. In-Vitro-Verfahren zum Screenen einer Substanz zur Vermeidung von Hautalterung in Verbindung mit der Menopause-Alterung, wobei das Verfahren die folgenden Schritte umfasst:
1) Behandeln von Probehautzellen mit einer Kandidatensubstanz zum Steuern der Expression eines Gens in den Zellen;
2) Exponieren der Probehautzellen an eine Umgebung mit einem Hautalterungsfaktor, um die Expression der Gene in den Zellen zu induzieren;
3) Messen der hautalterungsfaktor-induzierten Expression der Gene in den Zellen von Schritt 2); und
4) Identifizieren der Kandidatensubstanz als Substanz zur Vermeidung von Hautalterung, wenn sie die Expression eines Gens in den Zellen steuert,
wobei das Gen OGN ist,
wobei die Expression von OGN in der Haut von älteren Frauen im Vergleich zu jungen Frauen verringert ist.

## Revendications

1. Procédé *in vitro* pour cribler une substance destinée à prévenir le vieillissement cutané associé au vieillissement dû à la ménopause, le procédé comprenant les étapes consistant à :
1) traiter des cellules cutanées d'un échantillon avec une substance candidate pour contrôler l'expression d'un gène dans les cellules ;
2) exposer les cellules cutanées de l'échantillon à un environnement ayant un facteur de vieillissement cutané pour induire l'expression du gène dans les cellules;
3) mesurer l'expression induite par le facteur de vieillissement cutané du gène dans les cellules de l'étape 2) ; et
4) identifier la substance candidate comme une substance destinée à prévenir le vieillissement cutané si elle contrôle l'expression d'un gène dans les cellules,
dans lequel ledit gène est OGN,
dans lequel l'expression d'OGN est diminuée dans la peau des femmes âgées lorsque comparée aux jeunes femmes.
